# EUROPÄISCHE PATENTANMELDUNG

(11) **EP 2 946 730 A1**
(43) Veröffentlichungstag der Anmeldung: **25.11.2015**
(21) Anmeldenummer: 15164593.4
(22) Anmeldetag: 22.04.2015
(51) Int. Cl.: A61B 5/055

(54) **VERFAHREN ZUR VERMESSUNG DES ATEMVORGANGS EINES PATIENTEN WÄHREND EINER MAGNETRESONANZUNTERSUCHUNG, MESSANORDNUNG UND MAGNETRESONANZEINRICHTUNG**

(30) Priorität: 20.05.2014 DE 102014209488
(71) Anmelder: Siemens Aktiengesellschaft, 80333 München (DE)
(72) Erfinder: Biber, Stephan, 91056 Erlangen (DE); Fackelmeier, Andreas, 91177 Thalmässing (DE); Rehner, Robert, 91077 Neunkirchen am Brand (DE)

(57) **Zusammenfassung**

Verfahren zur Vermessung des Atemvorgangs eines Patienten (5) während einer Magnetresonanzuntersuchung mit einer Magnetresonanzeinrichtung (1), dadurch gekennzeichnet, dass die Reflektionseigenschaften wenigstens eines unterhalb des Patienten (5) angeordneten Spulenelements (6) vermessen und zur Ermittlung von den Atemzustand zu verschiedenen Zeitpunkten beschreibenden Atmungsdaten ausgewertet werden.

## Beschreibung

Die Erfindung betrifft ein Verfahren zur Vermessung des Atemvorgangs eines Patienten während einer Magnetresonanzuntersuchung, eine Messanordnung und eine Magnetresonanzeinrichtung.

Die Magnetresonanzbildgebung ist inzwischen insbesondere im medizinischen Bereich als etabliert anzusehen. Dabei wird ein Patient einem starken Magnetfeld, dem Grundmagnetfeld oder B0-Feld, ausgesetzt, so dass sich seine Spins ausrichten. Durch eine Hochfrequenzanregung können die ausgerichteten Spins dann angeregt werden und der Zerfall dieser Anregung wird als Magnetresonanzsignal gemessen. Gradientenfelder werden eingesetzt, um eine örtliche Auflösung zu erzielen.

Eine mögliche Fehlerquelle beziehungsweise Ursache für Artefakte bei Magnetresonanzuntersuchungen ist die Bewegung eines Patienten. Nachdem Magnetresonanzaufnahmen eine bestimmte Dauer in Anspruch nehmen ist es auch äußerst wichtig, dass der Patient über die gesamte Laufzeit der Magnetresonanzuntersuchung bewegungslos liegt. Bei Messungen im Abdomen-/Thoraxbereich ist diese Annahme nicht möglich, da das aufzunehmende Gewebe durch die Atmung des Patienten in jedem Fall Bewegungen unterworfen ist. Daher wurden bereits diverse Verfahren entwickelt, um aufgenommene Magnetresonanzdaten bezüglich der Atmung des Patienten, welche entsprechend vermessen wird, zu korrigieren. Ebenso wurde vorgeschlagen, Atmungsdaten des Patienten zum Triggern einer Messung einzusetzen. Werden Magnetresonanzdaten und parallel Atmungsdaten aufgenommen, können die Atmungsdaten auch ausgewertet werden, um nachträglich die Magnetresonanzdaten nach Atmungsphasen zu sortieren (retrospektives Gating).

Um den Atmungsverlauf des Patienten beschreibende Atmungsdaten zu erhalten, wurden im Stand der Technik bereits einige Möglichkeiten vorgeschlagen. So ist es beispielsweise bekannt, spezielle Magnetresonanzmessungen durchzuführen, insbesondere sogenannte Navigatoren. Dabei wird meist eine eindimensionale Messung im Bereich eines Kontrastübergangs, mithin einer Begrenzung zwischen unterschiedlichen Geweben, aufgenommen, so dass anhand der Verschiebung auf die Atmung rückgefolgert werden kann. Üblicherweise wird bei heutigen Navigatoren eine Begrenzung des Zwerchfells vermessen. Das Problem der Verwendung von Navigatoren ist deren Zeitaufwendigkeit, zudem ist durch die zusätzlichen Magnetresonanzmessungen eine höhere elektromagnetische Belastung des Patienten gegeben, das bedeutet, die SAR steigt. Jene ist jedoch meist beschränkt, was zu Einschränkungen bei der Gesamtuntersuchung führen kann.

Alternativ wurden zusätzliche Messeinrichtungen für die Atmung vorgeschlagen, insbesondere Atemgürtel und Atemkissen. Solche Messeinrichtungen werden um den Bauch beziehungsweise die Brust des Patienten gespannt oder zwischen einer anterioren Lokalspule und dem Bauch/der Brust des Patienten eingeklemmt. Zur korrekten Anordnung und Vorbereitung der Atemgürtel/Atemkissen ist ein erhöhter zusätzlicher Arbeitsaufwand erforderlich; zudem müssen zusätzlich zur Magnetresonanzeinrichtung externe Geräte mit eingesetzt werden.

Weitere Ansätze zur Vermessung der Atmung eines Patienten im Rahmen von Magnetresonanzuntersuchungen sind aus den ISMRM-Abstracts "An Alternative Concept of Non-sequenceinterfering, Contact-free-Respiration Monitoring", Proc. ISMRM 17 (2009) 753, und "An Alternative Concept of Non-Sequence-interfering Patient Respiration Monitoring", Proc. ISMRM 16 (2008) 202, bekannt. Dabei wird vorgeschlagen, die Atembewegung durch Beobachtung der Reflektionsparameter einer den gesamten Patienten zylinderförmig umschließenden Hochfrequenzspulenanordnung (body coil - BC) zu detektieren. Andere Ansätze, die sich mit der Beobachtung von Transmissionsparametern zwischen einer anterioren Lokalspule und einem Spulenelement hinter der Verkleidung der Patientenaufnahme der Magnetresonanzeinrichtung beschäftigen, sind aus der nachveröffentlichten deutschen Patentanmeldung DE 10 2013 212 276.4 der Anmelderin bekannt, die hiermit durch Bezugnahme in die hiesige Offenbarung mit aufgenommen wird.

Der Erfindung liegt damit die Aufgabe zugrunde, ein möglichst einfach realisierbares, robustes und verlässliches Verfahren zur Vermessung der Atmung eines Patienten während einer Magnetresonanzuntersuchung anzugeben.

Zur Lösung dieser Aufgabe wird erfindungsgemäß ein Verfahren zur Vermessung des Atemvorgangs eines Patienten während einer Magnetresonanzuntersuchung vorgesehen, welches sich dadurch auszeichnet, dass die Reflektionseigenschaften wenigstens eines unterhalb des Patienten angeordneten Spulenelements vermessen und zur Ermittlung von den Atemzustand zu verschiedenen Zeitpunkten beschreibenden Atmungsdaten ausgewertet werden.

Der erfindungsgemäße Ansatz beinhaltet also die Platzierung eines ortsfesten Spulenelements posterior zum Patienten im Thorax/Abdomenbereich des Patienten. Dabei ist das Spulenelement möglichst nahe am Patienten angeordnet, insbesondere weniger als 10 cm, bevorzugt weniger als 5 cm, von dem Körper des Patienten beabstandet. Wird nun über diese Antenne ein Sendesignal ausgesandt und das rücklaufende Signal als Messsignal vermessen, hängen dessen Charakteristika von den elektromagnetischen Eigenschaften des Patienten im Abdomen/Thoraxbereich ab, welche aber durch die Atmung verändert werden. Mithin kann durch Veränderung der hochfrequenztechnischen Eigenschaften des Spulenelements auf die Atembewegung rückgeschlossen werden. Es wird mithin für die Messung der elektromagnetische Effekt ausgenutzt, dass das magnetische Feld, welches von dem Spulenelement ausgesendet wird, an den menschlichen Körper ankoppelt und sich bis weit in den Körper hinein ausbreitet. Atmet der Patient beispielsweise ein, dehnt sich die Lunge aus und die Verteilung des Gewebes innerhalb des Empfindlichkeitsbereichs des Spulenelements verändert sich, so dass auch das in das Spulenelement, welches als Antenne wirkt, reflektierte Signal verändert wird.

Testmessungen haben gezeigt, dass auf diese Weise die Atemzyklen sowie auch auftretende Atempausen eindeutig erkennbar sind. Dabei können beispielsweise die Amplitude und/oder die Phase des rücklaufenden Signals von dem Spulenelement ausgewertet werden. Sehr geringe Sendeleistungen sind dabei bereits ausreichend, so dass beispielsweise vorgesehen sein kann, dass zur Messung der Reflektionseigenschaften eine Sendeleistung des Spulenelements kleiner als 10 dBm verwendet wird. Beispielsweise können - 10 dBm oder - 40 dBm verwendet werden. Somit liegt die Sendeleistung deutlich unter einem Milliwatt, was keine nennenswerte Belastung für den Patienten zur Folge hat und daher keinen beachtenswerten Einfluss auf das SAR liefert.

Dabei ist hervorzuheben, dass das hier beschriebene Verfahren sensitiv auf eine Veränderung der Gewebeverteilung und nicht nur auf die Ausdehnung des Körpers in anteriorer Richtung reagiert. Dies steht im Gegensatz zu Verfahren, die sensitiv auf die Abstandsänderung der Körperoberfläche zu einem weiter entfernten Sensor reagieren, beispielsweise bei Betrachtung der Transmissionen zwischen einer auf der Oberfläche des Patienten anterior positionierten Spule zu einem Spulenelement einer Ganzkörperspule, das hinter der Verkleidung der Patientenaufnahme der Magnetresonanzeinrichtung angeordnet ist. Somit ist das erfindungsgemäße Verfahren deutlich besser geeignet für sehr flachatmige (kranke) Patienten, deren Brustkorb beziehungsweise Bauch sich nur wenig ausdehnen. Das posterior angeordnete Spulenelement der vorliegenden Erfindung befindet sich in dem Bereich, in dem der Patient, insbesondere auf einer Patientenliege, aufliegt, so dass dort mithin keine Bewegung des Spulenelements auftritt, es sich jedoch dennoch gezeigt hat, dass die aufgrund der Nähe zum Patienten vermessbaren Änderungen der Gewebeverteilung beim Atmen ein hinreichend deutliches Signal liefern.

Es hat sich ferner gezeigt, nachdem das Spulenelement ja posterior zum Patienten im Abdomen- beziehungsweise Thoraxbereich angeordnet ist, Störungen, die durch Armbewegungen entstehen können, reduziert werden, da die Arme durch die dielektrischen Eigenschaften des Körpers selber, insbesondere des Torsos, abgeschirmt werden, was bei einer beispielsweise hinter einer Verkleidung einer Patientenaufnahme angeordneten Spule nicht der Fall wäre.

Mithin bietet die Verwendung eines posterior, also unterhalb des Patienten, nahe am Patienten im Abdomen- und/oder Thoraxbereich angeordneten Spulenelements zur Vermessung der Atmung eine Vielzahl von Vorteilen insbesondere auch gegenüber dem nachveröffentlichten Stand der Technik in Form der DE 10 2013 212 276.4. Dabei wird nicht eine Transmission zwischen unterschiedlichen Spulenelementen gemessen, sondern das als Antenne wirkende Spulenelement ist als ein Ein-Tor-Sensorelement zu verstehen.

Eine besonders bevorzugte Ausstattung der vorliegenden Erfindung sieht dabei vor, dass als Spulenelement wenigstens ein in eine Patientenliege der Magnetresonanzeinrichtung integriertes Spulenelement und/oder ein in eine posterior zum Patienten anzuordnende Lokalspule integriertes Spulenelement verwendet wird. Dies hat den großen Vorteil, dass zum einen die Nähe zum Patienten letztlich zwangsläufig hergestellt wird, zum anderen aber zur Vermessung der Atmung keine zusätzlichen Komponenten/Vorrichtungen mehr benötigt werden. Das Spulenelement ist bereits in die Patientenliege und/oder in die posterior, also unter dem Patienten, zu positionierende Lokalspule integriert, welche ohnehin verwendet wird. Die Lokalspule kann dabei insbesondere eine Wirbelsäulen-Lokalspule sein. In dieser Ausgestaltung verfährt sich der Sensor, also das Spulenelement, zusammen mit dem Patienten, wenn die Patientenliege in die Patientenaufnahme eingefahren oder aus der Patientenaufnahme herausgefahren wird, da es entweder in der bereits platzierten Lokalspule oder im beweglichen Teil des Patiententisches, der Patientenliege, selbst platziert ist. Daraus ergibt sich, dass die Atmungsdaten bereits sofort nach der Positionierung des Patienten außerhalb der Patientenaufnahme erfasst werden können. Ein Benutzer hat die Möglichkeit, sofort zu überprüfen, ob die Vermessung der Atmung des Patienten funktioniert und nicht erst, wenn sich der Patient bereits in der Patientenaufnahme befindet, wie dies bei Sensoren in der Patientenaufnahme oder der Transmission von einer anterioren Spule zu einem Sensor in der Patientenaufnahme der Fall wäre.

Ein weiterer Vorteil dieser Ausgestaltung, in der das Spulenelement in die Patientenliege oder eine unter dem Patienten zu legende Lokalspule integriert ist, ist, dass der Abstand zwischen dem Spulenelement und dem Patienten immer gleich bleibt, so dass sich die Wertebereiche der Kenndaten der Reflektionseigenschaften sich für unterschiedliche Patiententypen (schlank, adipös, muskulös) nur wenig verändern, was wiederum im Gegensatz zu Verfahren steht, bei denen ein hinter der Verkleidung der Patientenaufnahme angeordneter Sensor/Anteil einer Hochfrequenzspulenanordnung (Body coil) verwendet wird.

In konkreter Ausgestaltung des erfindungsgemäßen Verfahrens kann vorgesehen sein, dass ein nach einem Sendesignal rücklaufendes Signal mittels eines Richtkopplers abgetrennt und vermessen wird. Das bedeutet, es wird über das Spulenelement ein Sendesignal ausgegeben, welches als Reflektionssignal in das Spulenelement rückkoppelt und mithin ein rücklaufendes Signal erzeugt, welches als Messsignal durch einen Richtkoppler abgetrennt und vermessen wird. Insbesondere kann also eine Sende-/Empfangseinheit vorgesehen sein, die eine Sendeeinrichtung zum Aussenden eines Mess-Sendesignals, einen Richtkoppler zum Abtrennen eines rücklaufenden Signals und eine dem Richtkoppler nachgeordnete Empfangseinrichtung aufweisen kann.

Zweckmäßigerweise werden die Reflektionseigenschaften in Form der Amplitude und/oder Phase des rücklaufenden Signals ausgewertet. Dabei wird bevorzugt der Betrag der Amplitude des rücklaufenden Signals betrachtet. Beide Verläufe zeigen, wie Experimente der Anmelderin gezeigt haben, deutlich die Atembewegung an. Bevorzugt können sowohl die Amplitude als auch die Phase ausgewertet werden, um gegebenenfalls eine Plausibilisierung durchführen zu können und dergleichen.

Bevorzugt wird die Messung der Reflektionseigenschaften bei einer außerhalb des für die Magnetresonanzbildgebung genutzten Frequenzbereichs liegenden Frequenz durchgeführt. Dabei kann die Frequenz konkret in einem Bereich von 0,1 - 10000 MHz gewählt werden, beispielsweise also bei 20 MHz oder bei 250 MHz. Die Wahl der Messfrequenz für die Reflektionseigenschaften erfolgt mithin so, dass die Magnetresonanzbildgebung nicht gestört wird. Mithin werden die Frequenzen genutzt, bei denen das Empfangssystem der Magnetresonanzeinrichtung sehr unempfindlich ist. Dabei sei jedoch angemerkt, dass es durchaus denkbar ist, auch Frequenzen nahe der Magnetresonanzfrequenz (Larmorfrequenz) zu nutzen, insbesondere dann, wenn das Spulenelement auch bei der eigentlichen Magnetresonanzbildgebung als Empfangsspule eingesetzt wird, worauf im Folgenden noch näher eingegangen werden wird.

Wie bereits erwähnt wurde, können die zur Messung der Reflektionseigenschaften eingesetzten Leistungen äußerst gering gehalten werden, so dass beispielsweise vorgesehen sein kann, dass zur Messung der Reflektionseigenschaften eine Sendeleistung des Spulenelements kleiner als 10 dBm verwendet wird. So ist keine nennenswerte SAR-Belastung des Patienten zu berücksichtigen. Außerdem sind dann keine Störungen, beispielsweise Sättigung, der empfindlichen Empfangselektronik benachbarter Empfangsspulen zu erwarten.

Eine besonders zweckmäßige Ausgestaltung der Erfindung sieht vor, dass, insbesondere bei in die Patientenliege der Magnetresonanzeinrichtung integrierten Spulenelementen, wenigstens zwei an unterschiedlichen Positionen angeordnete Spulenelemente verwendet werden, wobei das zur Messung verwendete Spulenelement aufgrund einer die Lage des Patienten relativ zu den Spulenelementen, insbesondere die Lage des Patienten auf der Patientenliege, beschreibenden Patientenlageinformation und/oder einer die Qualität der Atmungsdaten der verschiedenen Spulenelemente beschreibenden Qualitätsinformation ausgewählt wird. Es kann mithin vorgesehen werden, mehr als ein einziges Spulenelement, also mehr als einen Sensor, zu verwenden, um flexibel auf andere Positionierungen des Patienten reagieren zu können und/oder unabhängiger von der Körpergröße des Patienten zu werden. So sind beispielsweise sowohl Magnetresonanzuntersuchungen bekannt, bei denen der Patient in der Ausrichtung "head first" positioniert wird, als auch solche, bei denen der Patient in der Ausrichtung "feet first" auf der Patientenliege angeordnet ist. Werden nun beispielsweise auf unterschiedlichen Seiten der Patientenliege integrierte Spulenelemente eingesetzt, kann immer das passende Spulenelement eingesetzt werden, wenn beispielsweise die Patientenlageinformation entsprechendes angibt. Die Auswahl des Spulenelements kann jedoch auch über die Analyse der Atmungsdatenqualität erfolgen, das bedeutet, es wird in einem Auswahlschritt zunächst mit allen Spulenelementen gemessen und dann das Spulenelement für die endgültige Messung ausgewählt, bei dem die höchste Signalqualität vorliegt.

Die Patientenlageinformation kann Patientendaten, insbesondere die Größe und/oder das Geschlecht und/oder das Gewicht und/oder das Alter des Patienten, umfassen und/oder wenigstens teilweise aus diesen abgeleitet werden. Es werden mithin Meta-Informationen genutzt, um herauszufinden, welches Spulenelement sich in der idealen Position zur Vermessung der Atmung des Patienten befindet. Zusätzlich oder alternativ kann jedoch auch vorgesehen sein, dass die Patientenlageinformation wenigstens teilweise aus einer Scoutmessung mit der Magnetresonanzrichtung ermittelt wird. In diesem Bereich sind auch bereits automatische Auswertungsverfahren bekannt, die mithin vollautomatisch ermitteln, ob der Patient "head first" oder "feet first" auf der Patientenliege angeordnet ist und auch wo er sich auf der Patientenliege befindet. Schließlich ist es selbstverständlich auch denkbar, dass die Patientenlageinformation eingegeben wird oder gar mittelbar ein geeignetes Spulenelement durch einen Bediener ausgewählt wird.

Eine zweckmäßige Ausgestaltung der vorliegenden Erfindung sieht vor, dass das Spulenelement, insbesondere eine Teilspule einer Lokalspule, auch zur Aufnahme von Magnetresonanzdaten verwendet wird. Wird das Spulenelement auch im Rahmen der Magnetresonanzbildgebung genutzt, beispielsweise, da es in eine bildgebende posteriore Lokalspule integriert ist, entsteht der Vorteil, dass keine zusätzliche Atemsensorantenne benötigt wird.

Dabei kann zum einen vorgesehen sein, dass das Spulenelement doppelresonant bei der Frequenz für eine Vermessung der Reflektionseigenschaften und bei der Magnetresonanzfrequenz betrieben wird. Dann ist mithin zum einen eine Resonanz bei der Larmorfrequenz (Magnetresonanzfrequenz) gegeben, zum anderen aber bei einer Atmungssensor-Frequenz, bei der die Messung der Reflektionseigenschaften vorgenommen wird.

Vorteilhaft kann es jedoch auch sein, wenn alternativ die Messung der Reflektionseigenschaften bei einer einem Rauschband für die Magnetresonanzbildgebung benachbarten Frequenz und/oder einer von einer Empfangseinrichtung für Magnetresonanzsignale verarbeitbaren Frequenz erfolgt. Die Frequenz zur Messung der Reflektionseigenschaften wird in dieser Ausgestaltung mithin zwar nahe der Magnetresonanzfrequenz (Larmorfrequenz) gewählt, bevorzugt gerade außerhalb des dort auftretenden Rauschbandes, aber so, dass die Messung nicht gestört wird, was es mit besonderem Vorteil ermöglichen kann, dass ein ohnehin bestehender Empfangskanal im Empfangssystem der Magnetresonanzeinrichtung, insbesondere also eine dortige Empfangseinrichtung, verwendet werden kann. Sowohl das die Reflektionseigenschaften beschreibende Signal als auch das Vermessen der Magnetresonanzsignale werden dann zunächst an dieselbe Empfangseinrichtung weitergeleitet, welcher die Signale entsprechend aufteilt und ihren jeweiligen Auswerteeinrichtungen zuführt.

Aufgrund der geringen Leistungen ist es zwar grundsätzlich denkbar, eine fast kontinuierliche Messung der Atmung vorzunehmen, wobei sich jedoch auch eine zyklische und/oder getriggerte Messung der Reflektionseigenschaften anbieten kann. In diesem Kontext kann vorgesehen sein, dass ein Messvorgang der Reflektionseigenschaften so getriggert wird, dass der Messzeitraum außerhalb eines Auslesezeitraums und/oder eines Anregungszeitraums eines Magnetresonanzbildgebungsvorgangs liegt. Dann kann ein Einfluss auf die Magnetresonanzbildgebung weitgehend ausgeschlossen werden, wobei jedoch zu beachten ist, dass dann für Auslesezeitfenster der Magnetresonanzfrequenz und der gleichen keine Atmungsdaten vorliegen. Daher kann es zweckmäßiger sein, die Frequenz, bei der die Reflektionseigenschaften gemessen werden, geschickt so zu wählen, dass eine möglichst geringe Beeinflussung der Magnetresonanzbildgebung auftritt.

In diesem Zusammenhang sieht eine andere vorteilhafte Ausgestaltung der vorliegenden Erfindung vor, dass das Spulenelement einen Sperrkreis bei der Magnetresonanzfrequenz umfasst. Das bedeutet, dass auf diese Weise die Messung der Reflektionseigenschaften auch nicht durch die zumindest zeitweise parallel ablaufende Magnetresonanzbildgebung beeinflusst wird, da entsprechende Signale innerhalb des Spulenelements vermieden werden können. Umgekehrt kann auch die Messung der Reflektionseigenschaften die Magnetresonanzbildgebung vorteilhaft nicht stören. Im Empfangsfall werden Verkopplungen, also das Mitschwingen, mit den bei der Magnetresonanzbildgebung verwendeten Spulenelementen minimiert. Diese Ausgestaltung ist selbstverständlich dann am zweckmäßigsten, wenn das Spulenelement dediziert für die Messung der Reflektionseigenschaften vorgesehen ist, mithin nicht für die Aufnahme von Magnetresonanzsignalen dient. In einem derartigen Fall kann das Spulenelement unabhängig von den Bildgebungsmaßnahmen in der Magnetresonanzuntersuchung genutzt werden.

Neben dem Verfahren betrifft die Erfindung auch eine Messanordnung zur Vermessung des Atemvorgangs eines Patienten während einer Magnetresonanzuntersuchung, aufweisend wenigstens ein bei zur Bildgebung positioniertem Patienten unterhalb des Patienten (also posterior) angeordnetes Spulenelement und eine Steuereinrichtung zur Aufnahme von die Reflektionseigenschaften des Spulenelements beschreibenden Messdaten und zur Ermittlung von den Atemzustand des Patienten zu verschiedenen Zeitpunkten beschreibenden Atmungsdaten durch Auswertung der Messdaten. Sämtliche Ausführungen bezüglich des erfindungsgemäßen Verfahrens lassen sich analog auf die erfindungsgemäße Messanordnung übertragen, so dass zweckmäßigerweise die Steuereinrichtung zur Durchführung des erfindungsgemäßen Verfahrens ausgebildet sein kann. Auf diese Weise werden auch mit der Messanordnung die Vorteile des erfindungsgemäßen Verfahrens erhalten. Besonders bevorzugt ist es in diesem Zusammenhang, wenn das wenigstens eine Spulenelement in die Patientenliege einer Magnetresonanzeinrichtung und/oder eine Lokalspule integriert ist.

Die Steuereinrichtung kann eine Sendeeinrichtung zum Aussenden eines Mess-Sendesignals, einen Richtkoppler zum Abtrennen eines rücklaufenden Signals, eine dem Richtkoppler nachgeschaltete Empfangseinrichtung und eine Auswerteeinrichtung umfassen. Die Sendeeinrichtung, der Richtkoppler und die Empfangseinrichtung können als eine Sende- und Empfangseinheit kombiniert werden. Die Auswerteeinrichtung kann Teil einer allgemeinen Recheneinrichtung sein.

Die Steuereinrichtung kann ferner eine Triggereinrichtung zum Triggern von Messvorgängen umfassen. Dabei können Messvorgänge für die Reflektionseigenschaften beispielsweise zyklisch wiederholt werden, es ist jedoch auch denkbar, diese von anderen eintretenden Ereignissen, beispielsweise bestimmten Ereignissen im Ablauf der Magnetresonanzuntersuchung, abhängig zu machen.

Schließlich betrifft die Erfindung auch eine Magnetresonanzeinrichtung, aufweisend eine erfindungsgemäße Messanordnung. Sämtliche Ausführungen zur erfindungsgemäßen Messanordnung und zum erfindungsgemäßen Verfahren lassen sich analog auf die erfindungsgemäße Magnetresonanzeinrichtung übertragen, bei der besonders bevorzugt das wenigstens eine Spulenelement in die Patientenliege und/oder eine Lokalspule integriert ist.

Weitere Vorteile und Einzelheiten der vorliegenden Erfindung ergeben sich aus den im Folgenden beschriebenen Ausführungsbeispielen sowie anhand der Zeichnung. Dabei zeigen:
- Fig. 1: eine Prinzipskizze einer erfindungsgemäßen Magnetresonanzeinrichtung,
- Fig. 2: Komponenten einer erfindungsgemäßen Messanordnung,
- Fig. 3: die Anordnung eines Spulenelements relativ zu einem Patienten,
- Fig. 4: eine gemessene Amplitude eines rücklaufenden Signals bei einem ersten Patienten,
- Fig. 5: eine gemessene Phase des rücklaufenden Signals bei dem ersten Patienten,
- Fig. 6: eine gemessene Amplitude des rücklaufenden Signals bei einem zweiten Patienten,
- Fig. 7: eine gemessene Phase des rücklaufenden Signals bei dem zweiten Patienten,
- Fig. 8: ein in eine Lokalspule integriertes Spulenelement, und
- Fig. 9: eine Patientenliege mit mehreren Spulenelementen.

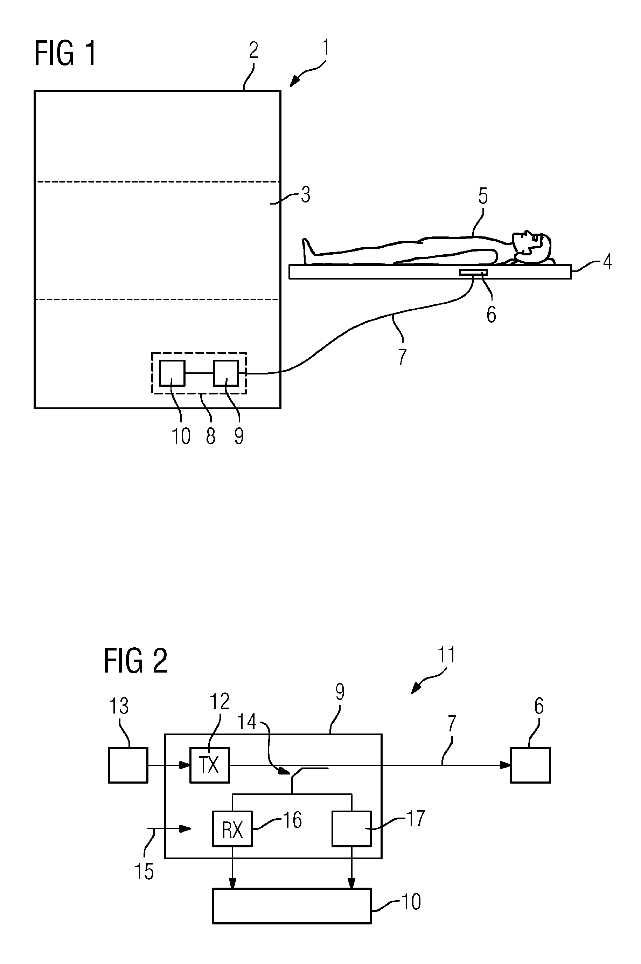

Fig. 1 zeigt eine Prinzipskizze einer erfindungsgemäßen Magnetresonanzeinrichtung 1. Diese weist, wie grundsätzlich bekannt, eine Hauptmagneteinheit 2 auf, die, hier gestrichelt angedeutet, eine zylindrische Patientenaufnahme 3 definiert. Die Patientenaufnahme 3 umgebend können, wie grundsätzlich bekannt, eine Hochfrequenzspulenanordnung (body coil) und eine Gradientenspulenanordnung vorgesehen sein; der das Grundmagnetfeld erzeugende Hauptmagnet ist ebenso in der Hauptmagneteinheit 2 enthalten.

Mittels einer Patientenliege 4 kann ein auf der Patientenliege 4 positionierter Patient 5 in die Patientenaufnahme 3 eingefahren werden, um eine Magnetresonanzuntersuchung durchzuführen, also Magnetresonanzbildgebung zu betreiben. Findet diese Magnetresonanzbildgebung im Bereich des Abdomens und/oder Thorax des Patienten 5 statt, ist zu beachten, dass dieser Bereich der Atmungsbewegung des Patienten 5 unterworfen ist. Daher soll auch vorliegend die Atmung des Patienten 5 vermessen werden, wozu die Magnetresonanzeinrichtung 1 eine entsprechende Messanordnung, konkret eine erfindungsgemäße Messanordnung, aufweist. Diese umfasst ein in die Patientenliege 4 integriertes Spulenelement 6, welches mithin posterior vom Patienten in dessen Abdomen- und/oder Thoraxbereich angeordnet ist. Das Spulenelement 6 ist durch die Positionierung innerhalb der Patientenliege 4 auch sehr nahe am Rücken des Patienten 5 angeordnet, insbesondere weniger als 5 cm davon entfernt. Es ist bezüglich des Patienten 5 fest positioniert, so dass auch bereits bei außerhalb der Patientenaufnahme 3 befindlichem Patienten 5 Messungen, beispielsweise zur Überprüfung der Funktion, vorgenommen werden können. Über eine Kabelverbindung 7, hier ein Koaxialkabel aus Kupfer, ist das Spulenelement 6 mit einer nur schematisch gezeigten Steuereinrichtung 8 der Magnetresonanzeinrichtung 1 verbunden, welche neben dem Betrieb der Magnetresonanzeinrichtung 1 selbst auch den Betrieb der Messanordnung gemäß dem erfindungsgemäßen Verfahren steuert, mithin als Steuereinrichtung 8 der Messanordnung wirkt. Hierzu umfasst die Steuereinrichtung 8 insbesondere eine Sende-/Empfangseinheit 9 und eine Auswerteeinrichtung 10.

Dies ist durch die Prinzipskizze der erfindungsgemäßen Messanordnung 11 in Fig. 2 näher erläutert. Die Sende-/Empfangseinheit 9 umfasst entsprechend zunächst eine Sendeeinrichtung 12, in der ein Mess-Sendesignal erzeugt wird, das über das Spulenelement 6 ausgegeben wird. Dies kann zyklisch geschehen, wozu die Sendeeinrichtung 12 das Signal eines Taktgebers 13 empfängt.

Wird das Mess-Sendesignal über das Spulenelement 6 in den Körper des Patienten 5 eingestrahlt, wird es durch die Eigenschaften der Gewebe, deren Position jedoch durch die Atmung verändert wird, beeinflusst, so dass abhängig vom Atmungszustand jeweils unterschiedliche rücklaufende Signale entstehen. Diese rücklaufenden Signale beschreiben die Reflektionseigenschaften. Sie werden durch einen Richtkoppler 14 abgetrennt und einer hier zweiteiligen Empfangseinrichtung 15 zugeführt, in der zum einen in einer Amplitudeneinheit 16 die Amplitude des rücklaufenden Signals digitalisiert wird, zum anderen in einer Phaseneinheit 17 durch Vergleich mit dem Sendesignal die aufgetretene Phasenverschiebung bestimmt und ebenso digitalisiert wird. Diese Messdaten, also die Amplitude und die Phase des rücklaufenden Signals, werden an die Auswerteeinrichtung 10 weitergeleitet. Diese werten den Amplitudenverlauf und den Phasenverlauf aus, um die Atmungsphasen zu verschiedenen Zeitpunkten beschreibende Atmungsdaten zu erzeugen, die von der Steuerungseinrichtung 8 beispielsweise zur Triggerung von Magnetresonanzmessungen und/oder zum retrospektiven Gating verwendet werden können.

Es sei an dieser Stelle noch angemerkt dass bereits kleine Sendeleistungen ausreichend sind, um ein aussagekräftiges rücklaufendes Signal, das auch als Atemsignal bezeichnet werden kann, zu erhalten.

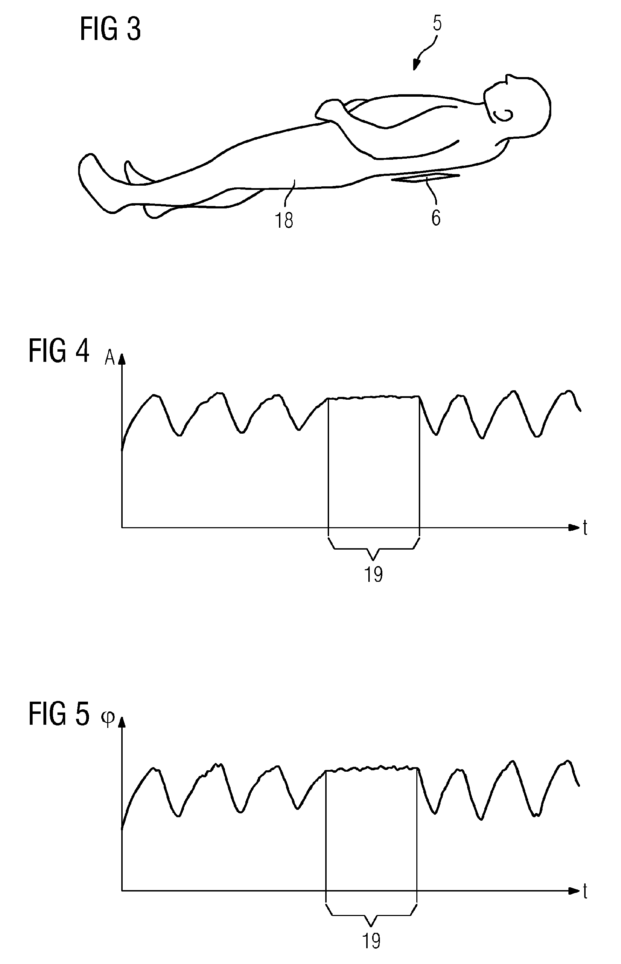

Fig. 3 zeigt zur Verdeutlichung eine perspektivische Ansicht zur Anordnung des Spulenelements 6 relativ zum Körper 18 des Patienten 5. Ersichtlich ist das Spulenelement 6 unterhalb, also posterior zum Körper 18 angeordnet, und zwar im mittleren Rückenbereich.

Es ist dabei grundsätzlich denkbar, das Spulenelement 6 auch zur Aufnahme von Magnetresonanzdaten im Rahmen der Magnetresonanzbildgebung zu nutzen. Dann kann das Spulenelement 6 doppelresonant ausgebildet sein, um ein Senden und Empfangen sowohl bei der Magnetresonanzfrequenz als auch bei der Frequenz zur Messung der Reflektionseigenschaften einzusetzen. Möglich ist es auch, die Messung der Reflektionseigenschaften bei einer Frequenz durchzuführen, die unmittelbar an einen Rauschbereich des zur Magnetresonanzbildgebung verwendeten Frequenzbereichs anschließt und so gegebenenfalls über denselben Empfangskanal ausgewertet werden kann, ohne dass eine Doppelresonanz erforderlich wäre.

Denkbar sind jedoch auch Ausführungsbeispiele, bei denen das Spulenelement 6 nicht zur Magnetresonanzbildgebung eingesetzt wird, mithin unabhängig von der Magnetresonanzbildgebung bei einer außerhalb des Frequenzbereichs für die Magnetresonanzbildgebung liegenden Frequenz betrieben werden kann. Messungen mit einem solchen Spulenelement sind durch die Figuren 4 - 7 beispielhaft dargestellt. Dabei betreffen die Figuren 4 und 5 einen ersten Patienten, wobei in Figur 4 der zeitliche Verlauf der Amplitude des rücklaufenden Signals dargestellt ist, in Figur 5 der zeitliche Verlauf der Phase des rücklaufenden Signals. Die Messung wurde bei einer Frequenz von 20 MHz durchgeführt. Die Atemzyklen sowie die Atempause in einem Zeitbereich 19 sind eindeutig erkennbar.

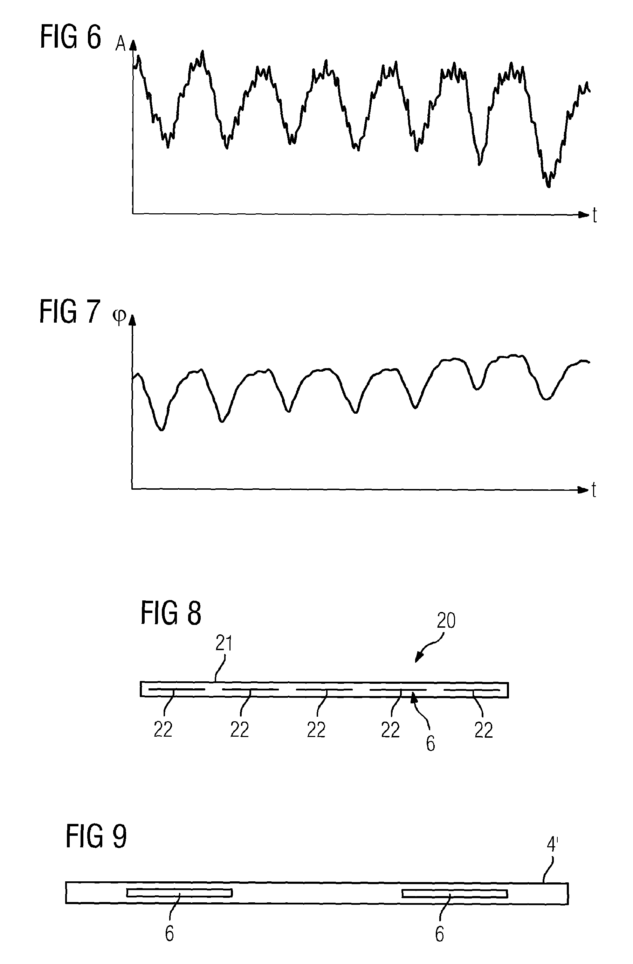

Die Figuren 6 und 7 zeigen weitere Messergebnisse für einen zweiten Patienten, wobei wiederum Fig. 6 (den Betrag der) Amplitude betrifft und Fig. 7 die Phase. Hier liegt keine Atempause vor. Nachdem es sich bei dem zweiten Patienten um eine sehr schlanke Frau handelt, lässt sich sogar der Herzschlag dem Atemsignal überlagert erkennen. Ähnliche Messergebnisse wie die der Figuren 4 - 7 wurden auch bei Frequenzen von 250 MHz erhalten.

Figur 8 zeigt eine Prinzipskizze einer Lokalspule 20, hier einer Wirbelsäulenspule, die unter den Patienten 5 auf die Patientenliege 4 gelegt werden kann. In einem flexiblen Trägermaterial 21 sind verschiedene Teilspulen 22 eingebettet. Eine günstig Positionierte dieser Teilspulen kann dabei ebenso als Spulenelement 6 für die Durchführung der vorliegenden Erfindung verwendet werden, so dass dann das Spulenelement 6 nicht in der Patientenliege 4, sondern in der Lokalspule 20 integriert ist.

Figur 9 zeigt schließlich eine modifizierte Ausgestaltung einer Patientenliege 4', in die symmetrisch an gegenüberliegenden Seiten zwei Spulenelemente 6 zur Vermessung der Atmung eines Patienten integriert sind. Auf diese Weise kann die Atmung des Patienten 5 in jedem Fall vermessen werden, unabhängig davon, ob der Patient "head first" oder "feet first" auf der Patientenliege 4' positioniert ist. Befindet sich der Kopf des Patienten 5 auf der rechten Seite der Patientenliege 4', wird auch das rechte Spulenelement 6 die besseren Ergebnisse für die Atmungsdaten liefern, im anderen Falle das linke Spulenelement 6. Mithin ist ein Spulenelement 6 auszuwählen, welches die Messergebnisse liefern soll. Dies kann in Abhängigkeit einer Patientenlageinformation bestimmt werden, die aus einer Scoutmessung und/oder, insbesondere bei einer größeren Vielzahl von Spulenelementen 6, Patientendaten, beispielsweise der Größe, dem Gewicht und dergleichen, ermittelt werden kann. Auch die Eingabe einer Patientenlageinformation ist selbstverständlich möglich, genau wie die unmittelbare Auswahl eines zu verwendenden Spulenelements 6. Die Auswahl des Spulenelements 6 kann auch in Abhängigkeit einer Qualitätsinformation getroffen werden, zu deren Ermittlung Testmessungen bei positioniertem Patienten 5 mit allen Spulenelementen 6 durchgeführt werden und das Spulenelement 6 verwendet wird, welches die qualitativ hochwertigsten Messdaten beziehungsweise Atmungsdaten liefert. Diese Auswahltätigkeit kann auch automatisiert über die Steuereinrichtung 8 erfolgen.

Selbstverständlich können dabei auch mehr als zwei Spulenelemente verwendet werden.

Es sei in diesem Kontext noch angemerkt, dass das hier vorgeschlagene Messverfahren auch in Ergänzung zu einer bestehenden Methode, beispielsweise der Verwendung eines Atemkissens oder eines Atemgürtels, verwendet werden kann. Auch in einem solchen Fall können der Bediener und/oder die Steuereinrichtung 8 entscheiden, welches Messverfahren im konkreten Fall eingesetzt wird. Beispielsweise kann als Grundeinstellung eine Messung über das Spulenelement 6 erfolgen, wobei nur dann, wenn ein weiterer Sensor, beispielsweise ein Atemkissen, angeschlossen wurde, dieses mit höherer Priorität ausgewertet werden kann.

Schließlich wird darauf hingewiesen, dass insbesondere bei Spulenelementen 6, die nicht zur Messung von Magnetresonanzsignalen eingesetzt werden, zweckmäßigerweise ein Sperrkreis für die Magnetresonanzsequenz Teil des Spulenelements sein kann, um eine Störung der Atmungsvermessung durch die Magnetresonanzbildgebung und umgekehrt zu vermeiden oder zu minimieren.

Obwohl die Erfindung im Detail durch das bevorzugte Ausführungsbeispiel näher illustriert und beschrieben wurde, so ist die Erfindung nicht durch die offenbarten Beispiele eingeschränkt und andere Variationen können vom Fachmann hieraus abgeleitet werden, ohne den Schutzumfang der Erfindung zu verlassen.

## Patentansprüche

1. Verfahren zur Vermessung des Atemvorgangs eines Patienten (5) während einer Magnetresonanzuntersuchung mit einer Magnetresonanzeinrichtung (1), **dadurch gekennzeichnet, dass** die Reflektionseigenschaften wenigstens eines unterhalb des Patienten (5) angeordneten Spulenelements (6) vermessen und zur Ermittlung von den Atemzustand zu verschiedenen Zeitpunkten beschreibenden Atmungsdaten ausgewertet werden.

2. Verfahren nach Anspruch 1, **dadurch gekennzeichnet, dass** als Spulenelement (6) wenigstens ein in eine Patientenliege (4, 4') der Magnetresonanzeinrichtung (1) integriertes Spulenelement (6) und/oder ein in eine posterior zum Patienten (5) anzuordnende Lokalspule (20) integriertes Spulenelement (6) verwendet wird.

3. Verfahren nach einem der vorangehenden Ansprüche, **dadurch gekennzeichnet, dass** ein nach einem Sendesignal rücklaufendes Signal mittels eines Richtkopplers (14) abgetrennt und vermessen wird.

4. Verfahren nach Anspruch 3, **dadurch gekennzeichnet, dass** die Reflektionseigenschaften in Form der Amplitude und/oder Phase des rücklaufenden Signals ausgewertet werden.

5. Verfahren nach einem der vorangehenden Ansprüche, **dadurch gekennzeichnet, dass** die Messung der Reflektionseigenschaften bei einer außerhalb des für die Magnetresonanzbildgebung genutzten Frequenzbereichs liegenden Frequenz durchgeführt wird.

6. Verfahren nach Anspruch 5, **dadurch gekennzeichnet, dass** die Frequenz in einem Bereich von 0,1 bis 10000 MHz gewählt wird.

7. Verfahren nach einem der vorangehenden Ansprüche, **dadurch gekennzeichnet, dass** zur Messung der Reflektionseigenschaften eine Sendeleistung des Spulenelements (6) kleiner als 10 dBm verwendet wird.

8. Verfahren nach einem der vorangehenden Ansprüche, **dadurch gekennzeichnet, dass**, insbesondere bei in die Patientenliege (4, 4') der Magnetresonanzeinrichtung (1) integrierten Spulenelementen (6), wenigstens zwei an unterschiedlichen Positionen angeordnete Spulenelemente (6) verwendet werden, wobei das zur Messung verwendete Spulenelement (6) aufgrund einer die Lage des Patienten (5) relativ zu den Spulenelementen (6), insbesondere die Lage des Patienten (5) auf der Patientenliege (4, 4'), beschreibenden Patientenlageinformation und/oder einer die Qualität der Atmungsdaten der verschiedenen Spulenelemente (6) beschreibenden Qualitätsinformation ausgewählt wird.

9. Verfahren nach Anspruch 8, **dadurch gekennzeichnet, dass** die Patientenlageinformation Patientendaten, insbesondere die Größe und/oder das Geschlecht und/oder das Gewicht und/oder das Alter des Patienten (5), umfasst und/oder wenigstens teilweise aus diesen abgeleitet wird.

10. Verfahren nach Anspruch 8 oder 9, **dadurch gekennzeichnet, dass** die Patientenlageinformation wenigstens teilweise aus einer Scoutmessung mit der Magnetresonanzeinrichtung (1) ermittelt wird.

11. Verfahren nach einem der vorangehenden Ansprüche, **dadurch gekennzeichnet, dass** das Spulenelement (6), insbesondere eine Teilspule (22) einer Lokalspule (20), auch zur Aufnahme von Magnetresonanzdaten verwendet wird.

12. Verfahren nach Anspruch 11, **dadurch gekennzeichnet, dass** das Spulenelement (6) doppelresonant bei der Frequenz für die Vermessung der Reflektionseigenschaften und bei der Magnetresonanzfrequenz ausgebildet ist.

13. Verfahren nach Anspruch 11, **dadurch gekennzeichnet, dass** die Messung der Reflektionseigenschaften bei einer einem Rauschband für die Magnetresonanzbildgebung benachbarten Frequenz und/oder einer von einer Empfangseinrichtung für Magnetresonanzsignale verarbeitbaren Frequenz erfolgt.

14. Verfahren nach einem der vorangehenden Ansprüche, **dadurch gekennzeichnet, dass** das Spulenelement (6) weniger als 10 cm, insbesondere weniger als 5 cm, von dem Körper (18) des Patienten (5) beabstandet ist.

15. Verfahren nach einem der vorangehenden Ansprüche, **dadurch gekennzeichnet, dass** ein Messvorgang der Reflektionseigenschaften so getriggert wird, dass der Messzeitraum außerhalb eines Auslesezeitraums und/oder eines Anregungszeitraums eines Magnetresonanzbildgebungsvorgangs liegt.

16. Messanordnung (11) zur Vermessung des Atemvorgangs eines Patienten (5) während einer Magnetresonanzuntersuchung mit einer Magnetresonanzeinrichtung (1), aufweisend wenigstens ein bei zur Bildgebung positioniertem Patienten (5) unterhalb des Patienten (5) positioniertes Spulenelement (6) und eine Steuereinrichtung (8) zur Aufnahme von die Reflektionseigenschaften des Spulenelements (6) beschreibenden Messdaten und zur Ermittlung von den Atemzustand des Patienten (5) zu verschiedenen Zeitpunkten beschreibenden Atmungsdaten durch Auswertung der Messdaten.

17. Messanordnung nach Anspruch 16, **dadurch gekennzeichnet, dass** die Steuereinrichtung (8) eine Sendeeinrichtung (12) zum Aussenden eines Mess-Sendesignals, einen Richtkoppler (14) zum Abtrennen eines rücklaufenden Signals, eine dem Richtkoppler (14) nachgeschaltete Empfangseinrichtung (15) und eine Auswerteeinrichtung (10) umfasst.

18. Messanordnung nach Anspruch 16 oder 17, **dadurch gekennzeichnet, dass** die Steuereinrichtung (8) ferner eine Triggereinrichtung zum Triggern von Messvorgängen umfasst.

19. Messanordnung nach einem der Ansprüche 16 bis 18, **dadurch gekennzeichnet, dass** das nicht bei der Magnetresonanzbildgebung zu verwendende Spulenelement (6) einen Sperrkreis bei der Magnetresonanzfrequenz umfasst.

20. Messanordnung nach einem der Ansprüche 16 bis 19, **dadurch gekennzeichnet, dass** die Steuereinrichtung (8) zur Durchführung eines Verfahrens nach einem der Ansprüche 1 bis 15 ausgebildet ist.

21. Magnetresonanzeinrichtung (1), aufweisend eine Messanordnung (11) nach einem der Ansprüche 16 bis 20.
